# EUROPEAN PATENT APPLICATION

(11) **EP 2 281 517 A1**
(43) Date of publication of application: **09.02.2011**
(21) Application number: 10251396.7
(22) Date of filing: 05.08.2010
(51) Int. Cl.: A61B 17/34

(54) **Surgical access device having non-circular cross-section**

(30) Priority: 06.08.2009 US 231781 P; 06.08.2009 US 231790 P; 06.08.2009 US 231798 P; 06.08.2009 US 231806 P; 11.06.2010 US 813838
(71) Applicant: Tyco Healthcare Group, LP, New Haven CT 06511 (US)
(72) Inventor: Kleyman, Gennady, Brooklyn, NY 11230 (US)
(74) Representative: Soames, Candida Jane

(57) **Abstract**

A surgical apparatus for positioning within a tissue tract accessing an underlying body cavity. The apparatus may include a seal anchor member comprising a compressible material. The seal anchor member may be adapted to transition between a first condition for insertion of at least a portion of the seal anchor member within a tissue tract and a second condition to facilitate a securing of the seal anchor member within a tissue tract and in substantial scaled relation with tissue surfaces defining a tissue tract. The seal anchor member may have proximal and distal ends and may define at least one port extending between the proximal and distal ends, the at least one port being adapted for the reception of an object whereby compressible material defining the at least one port is adapted to deform to establish a substantial scaled relation with the object. The seal anchor member may have a non-circular cross-section..

## Description

### CROSS REFERENCE TO RELATED APPLICATION

The present application claims the benefit of and priority to U.S. Provisional Application Serial No. 61/231,806 filed on August 6, 2009, the entire contents of which are incorporated herein by reference.

### BACKGROUND

### 1. Technical Field

The present disclosure relates to a seal for use in a surgical procedure. More particularly, the present disclosure relates to a seal anchor member adapted for insertion into an incision in tissue, and, for the scaled reception of one or more surgical objects such that a substantially fluid-tight seal is formed with both the tissue and the surgical object, or objects.

### 2. Background of the Related Art

Today, many surgical procedures are performed through small incisions in the skin, as compared to the larger incisions typically required in traditional procedures, in an effort to reduce both trauma to the patient and recovery time. Generally, such procedures are referred to as "endoscopic", unless performed on the patient's abdomen, in which case the procedure is referred to as "laparoscopic". Throughout the present disclosure, the term "minimally invasive" should be understood to encompass, e.g., endoscopic, laparoscopic, arthroscopic, thoracic procedures.

During a typical minimally invasive procedure, surgical objects, such as surgical access devices, e.g., trocar and cannula assemblies, or endoscopes, are inserted into the patient's body through the incision in tissue. In general, prior to the introduction of the surgical object into the patient's body, insufflation gases are used to enlarge the area surrounding the target surgical site to create a larger, more accessible work area. Accordingly, the maintenance of a substantially fluid-tight seal is desirable so as to inhibit the escape of the insufflation gases and the deflation or collapse of the enlarged surgical site.

To this end, various valves and seals are used during the course of minimally invasive procedures and are widely known in the art. However, a continuing need exists for a seal anchor member that can be inserted directly into an incision in tissue in a narrow area, such as a cavity between two ribs, and that can accommodate a variety of surgical objects while maintaining the integrity of an insufflated workspace.

### SUMMARY

According to an embodiment of the present invention, there is provided a surgical apparatus for positioning within a tissue tract accessing an underlying body cavity includes a seal anchor member comprising a compressible material and being adapted to transition between a first expanded condition and a second compressed condition. The first expanded condition facilitates a securing of the seal anchor member within the tissue tract and in substantial scaled relation with tissue surfaces defining the tissue tract, and the second compressed condition facilitates an at least partial insertion of the seal anchor member within the tissue tract. The seal anchor member may be formed of a foam material, which may be at least partially constituted of a material selected from the group consisting of polyisoprene, urethane, and silicone. Alternatively, the seal anchor member may be formed of a gel material.

The seal anchor member includes proximal and distal ends that defme elongated, e.g., oval or oblong, perimeters to facilitate the positioning of the seal anchor member within a tissue tract accessing an underlying body cavity. At least one of the proximal and distal ends of the seal anchor member may exhibit an arcuate configuration, which may be either concave or convex. The seal anchor member may be rolled, twisted, or otherwise deformed to fit nonlinearly into the tissue tract. The seal anchor member may also be cut to better suit a surgical procedure.

At least one port extends between the proximal and distal ends and is adapted for the reception of an object whereby compressible material defining the at least one port is adapted to deform to establish a substantial sealed relation with the object. The at least one port may contain at least an undercut lo protect against fluid leaks. The seal anchor member may include a plurality of ports that may be configured linearly with respect to the major diameter of the perimeter of at least one of the distal and proximal ends. Each port may be spaced equally from its neighboring ports.

According to an embodiment of the present invention, there is provided a surgical apparatus for positioning within a tissue tract accessing an underlying body cavity, which comprises: a seal anchor member comprising a compressible material. The seal anchor member may be adapted to transition between a first condition for insertion of at least a portion of the seal anchor member within a tissue tract and a second condition to facilitate a securing of the seal anchor member within a tissue tract and in substantial scaled relation with tissue surfaces defining a tissue tract. The seal anchor member may have proximal and distal ends and define at least one port extending between the proximal and distal ends, the at least one port being adapted for the reception of an object whereby compressible material defining the at least one port is adapted to deform to establish a substantial sealed relation with the object. The seal anchor member may have a non-circular cross-section.

The seal anchor member may be formed of a foam material. The foam material may be at least partially constituted of a material selected from the group consisting of polyisoprene, urethane, and silicone. The seal anchor member may also be formed of a gel material. The at least one port may include at least one undercut to reduce the likelihood of leaks therethrough. Also, the surgical apparatus may include indicia that indicates to a user a location at which the apparatus may be cut. The seal anchor member may include a plurality of ports, and the plurality of ports may be configured linearly with respect to each other. Each port of the plurality of ports may be spaced equally from its neighboring ports. In use, the seal anchor member may have an initial expanded condition, and may be adapted to be compressed by an external compressing force from the initial expanded condition to the first condition to facilitate insertion of at least a portion of the seal anchor member within a tissue tract, the anchor seal member being further adapted upon removal of the compressing force to expand towards its initial expanded condition and to its second condition to facilitate a securing of the seal anchor member within a tissue tract and in substantial sealed relation with tissue surfaces defining a tissue tract.

According to another embodiment of the present invention, there is provided a surgical apparatus for positioning within a tissue tract accessing an underlying body cavity, which comprises: a seal anchor member comprising a compressible material; the seal anchor member being adapted to transition between a first condition for insertion of at least a portion of the seal anchor member within a tissue tract and a second condition to facilitate a securing of the seal anchor member within a tissue tract and in substantial sealed relation with tissue surfaces defining a tissue tract, the seal anchor member having proximal and distal ends and defining a plurality of ports extending between the proximal and distal ends, at least one of the plurality of ports being adapted for the reception of an object whereby compressible material defining the at least one port is adapted to deform to establish a substantial sealed relation with the object, wherein the plurality of ports are arranged linearly relative to each other.

The seal anchor member may be formed of a foam material. The foam material may be at least partially constituted of a material selected from the group consisting of polyisoprene, urethane, and silicone. The seal anchor member may also be formed of a gel material. The port may include at least one undercut to reduce the likelihood of leaks therethrough. The surgical apparatus may include indicia that indicates to a user a location at which the apparatus may be cut. The seal anchor member may have a non-circular cross-section. Each port may be spaced equally from its adjacent ports. In use, the seal anchor member may have an initial expanded condition, and may be adapted to be compressed by an external compressing force from the initial expanded condition to the first condition to facilitate insertion of at least a portion of the seal anchor member within a tissue tract, the anchor seal member being further adapted upon removal of the compressing force to expand towards its initial expanded condition and to its second condition to facilitate a securing of the seal anchor member within a tissue tract and in substantial sealed relation with tissue surfaces defining a tissue tract.

According to still another embodiment of the present invention, there is provided a surgical apparatus for positioning within a tissue tract accessing an underlying body cavity, which comprises: a seal anchor member comprising a compressible material; the seal anchor member being adapted to transition between a first condition for insertion of at least a portion of the seal anchor member within a tissue tract and a second condition to facilitate a securing of the seal anchor member within a tissue tract and in substantial sealed relation with tissue surfaces defining a tissue tract, the seal anchor member having proximal and distal ends and defining at least one port extending between the proximal and distal ends, the at least one port being adapted for the reception of an object whereby compressible material defining the at least one port is adapted to deform to establish a substantial sealed relation with the object, and wherein the at least one port includes an undercut to reduce the likelihood of leaks therethrough.

According to still another embodiment of the present invention, there is provided a seal anchor member formed from a foam material and being elongated in cross-section, the member being configured and dimensioned to be compressed for insertion into an incision and, once inserted, to expand so as to be secured within and seal against the incision, the seal anchor member defining at least one port extending generally longitudinally and being adapted for sealed reception of a surgical object. When the seal anchor member is positioned within an incision, the seal anchor member may exert a biasing force against the incision. Advantageously, when positioned within an elongated incision, the biasing force exerted by the seal anchor member against the incision is substantially equal around a perimeter of the seal anchor member. The foam material may be at least partially constituted of a material selected from the group consisting of polyisoprene, urethane, and silicone. The seal anchor member may include a plurality of ports.

According to still another embodiment of the present invention, there is provided a seal anchor member formed from a foam material, the member being configured and dimensioned to be compressed for insertion into an elongated incision and, once inserted, to expand so as to exert a biasing force against the elongated incision, the seal anchor member having a shape in cross-section such that the biasing force exerted by the seal anchor member against the elongated incision is substantially equal around a perimeter of the seal anchor member. The seal anchor member may define at least one port extending generally longitudinally and being adapted for sealed reception of a surgical object. The foam material may be at least partially constituted of a material selected from the group consisting of polyisoprene, urethane, and silicone. The seal anchor member may include a plurality of ports. The seal anchor member may define a major diameter in a first direction and a minor diameter in a second direction that is perpendicular to the first direction, the major diameter being greater than the minor diameter. The seal anchor member may include an intermediate portion and an end portion, at least one of a major diameter and a minor diameter of the end portion being greater than a major diameter or a minor diameter of the intermediate portion so as to aid in retaining the seal anchor member within an incision.

### BRIEF DESCRIPTION OF THE DRAWINGS

Various embodiments of the present disclosure are described hereinbelow with references to the drawings, wherein:
**FIG. 1** is a top, perspective view of a surgical apparatus in accordance with the principles of the present disclosure shown in an expanded condition illustrating a seal anchor member positioned relative to the tissue;
**FIG. 2** is a side, schematic view of the seal anchor member of **FIG. 1**;
**FIG. 3** is a cross-sectional view of the seal anchor member of **FIG. 1** taken along section line 3-3 of **FIG. 1** illustrating a plurality of ports defining undercuts;
**FIG. 4** is a side, schematic view of a port of the seal anchor member of **FIG. 2** with a surgical object inserted therethrough;
**FIG. 5** is a perspective, schematic view of the seal anchor member of **FIG. 1** shown in a compressed condition prior to the insertion thereof into an incision in tissue;
**FIG. 6** is a perspective, schematic view of the seal anchor member of **FIG. 1** shown in the expanded condition and subsequent to its insertion into the incision;

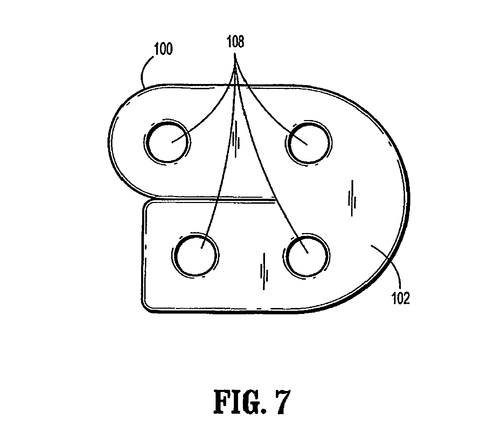

**FIG. 7** is a lop, plan view of the seal anchor member of **FIG. 1** in a rolled state; and
**FIGS. 8A-8D** are perspective views of a surgical apparatus in accordance with another embodiment of the present disclosure illustrating a seal anchor member cut to varying lengths.

### DETAILED DESCRIPTION OF THE EMBODIMENTS

In the drawings and in the description which follows, in which like references numerals identify similar or identical elements, the term "'proximal" will refer to die end of the apparatus which is closest to the clinician during use, while the term "distal" will refer to the end which is furthest from the clinician, as is traditional and known in the art.

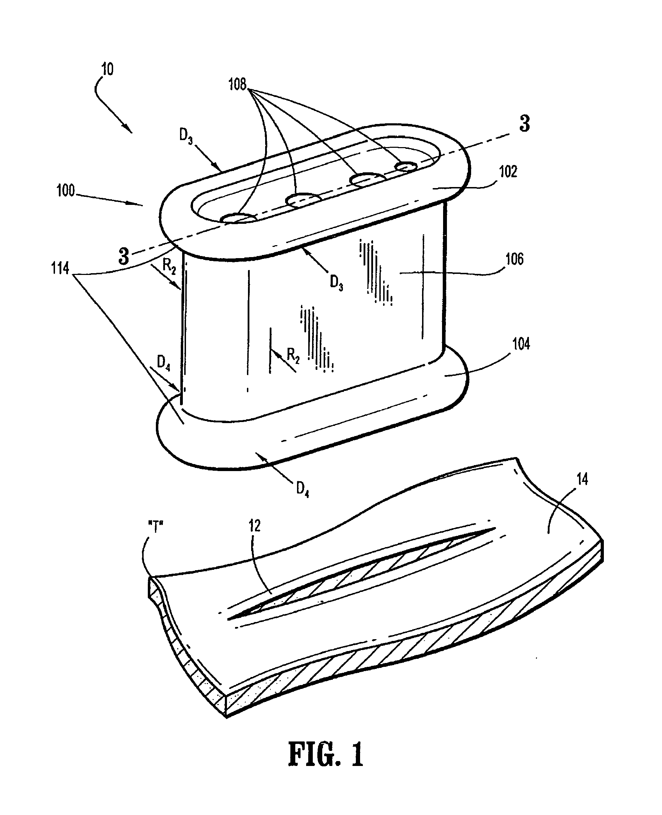

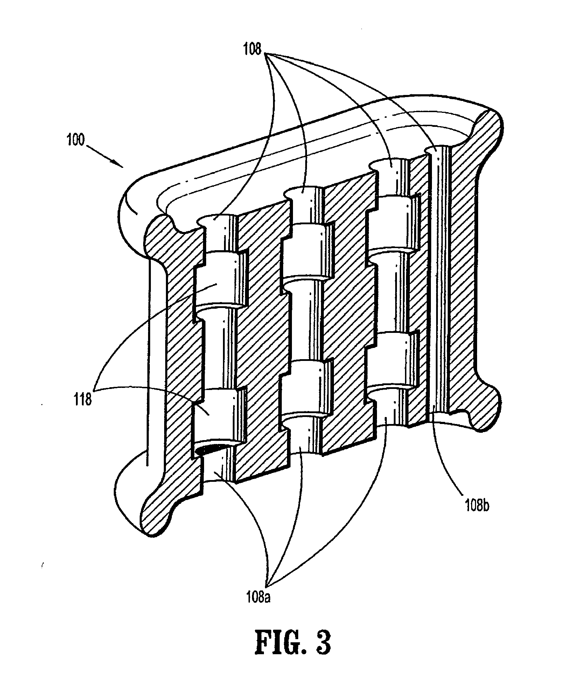

With reference to **FIGS.1-4****,** a surgical apparatus **10** for use in a surgical procedure, e.g., a minimally invasive procedure is illustrated. Surgical apparatus **10** includes seal anchor member **100** having proximal end **102** and distal end **104.** Seal anchor member **100** includes one or more ports **108,** i.e., lumen, that extend through seal anchor member **100** between proximal end **102** and distal end **104.**

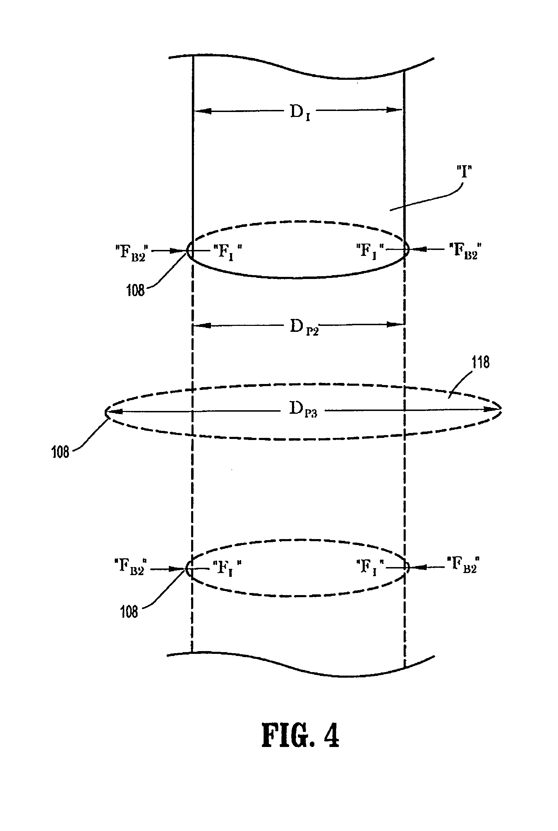

Seal anchor member **100** is formed from a suitable foam material having sufficient compliance to form a seal about one or more surgical objects, shown generally as surgical object **"I" (****FIG. 4****),** and also establish a scaling relation with tissue **"T".** The foam is sufficiently compliant to accommodate motion of the surgical object **"I".** In one embodiment, the foam includes a polyisoprene material. An example of an anchor member formed of, e.g., foam, is disclosed in applicant's co-pending U.S. Patent Application Serial Number 12/244,024, filed October 2, 2008, the entire contents of which are hereby incorporated by reference herein.

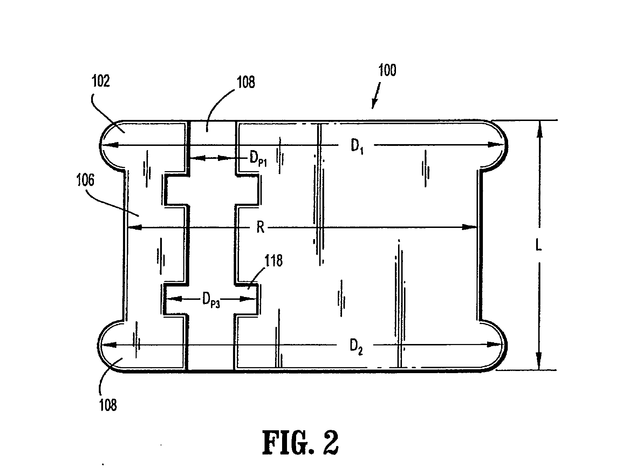

Proximal end **102** of seal anchor member **100** defines a first major diameter **D₁** and distal end **104** defines a second major diameter **D₂.** In an embodiment of seal anchor member **100,** the respective first and second major diameters **D₁, D₂** of the proximal and distal ends **102,104** are substantially equivalent, as seen in **FIG. 2****,** although an embodiment of seal anchor member **100** in which diameters **D₁, D₂** are different is also within the scope of the present disclosure. Also, proximal end **102** of seal anchor member **100** defines a first minor diameter **D₃** and distal end **104** defines a second minor diameter **D₄.** In an embodiment of seal anchor member **100,** the respective first and second minor diameters **D₃, D₄** of the proximal and distal ends **102, 104** are substantially equivalent, as seen in **FIG. 2****,** although an embodiment of seal anchor member **100** in which diameters **D₃, D₄** are different is also within the scope of the present disclosure. Advantageously, first and second major diameters **D₁, D₂** of the proximal and distal ends **102, 104** are greater than first and second minor diameters **D₃, D₄** of the proximal and distal ends **102, 104,** such that the seal anchor member **100** has, in cross-section, a non-circular, e.g., oblong, oval, race-track, etc., shape.

As depicted in **FIG. 1****,** positioning members **114** of proximal and distal ends **102,104** may define arcuate surfaces to assist in the insertion of seal anchor member **100** within a tissue tract **12** defined by tissue surfaces **14** and formed in tissue **"T",** e.g., an incision, as discussed in further detail bellow. Alternatively, proximal and distal ends **102, 134** may define substantially planar surfaces or substantially arcuate surfaces. Embodiments are contemplated herein in which either or both of proximal and distal ends **102, 104** define surfaces that are either or both arcuate or planar. The arcuate surfaces may be either or both concave or convex.

Intermediate portion **106** extends between proximal and distal ends **102, 104** to define a dimension, or length, **"L"** therealong. Intermediate portion **106** further defines an intermediate major diameter **"R"** substantially parallel to major diameters **D₁, D₂.** The dimension **"R"** of intermediate portion **106** may remain substantially uniform along the dimension **"L"** thereof. Alternatively, the dimension **"R"** of intermediate portion **106** may vary along the dimension, or length, **"L"** thereof, thereby defining a cross-sectional dimension that varies along its length **"L",** which facilitates the anchoring of seal anchor member **100** within tissue **"T".** In addition. intermediate portion **106** may further define an intermediate minor diameter **"R₂"** substantially perpendicular to major diameter **R.** Advantageously, the intermediate minor diameter **"R₂"** may be smaller than the major diameter **R,** such that the seal anchor member **100** has, in cross-section, a non-circular, e.g., oblong, oval, race-track, etc., shape.

The dimension **"R"** of intermediate portion **106** may be appreciably less than the respective major axes **D₁, D₂** of proximal and distal ends **102, 104** to assist in anchoring seal anchor member **100** within tissue **"T"** as discussed in further detail below. However, in an alternate embodiment, the dimension **"R"** of intermediate portion **106** may be substantially equivalent to the respective major axes **D₁, D₂** of proximal and distal ends **102,104.** In cross section, intermediate portion 106 may exhibit any suitable elongated configuration, e.g., substantially oval or oblong, for insertion into a narrow incision.

Each port **108** is configured to removably receive the surgical object **"I".** Prior to the insertion of surgical object **"I",** port **108** is in a first state in which port **108** defines a first or initial dimension **D_{P1}.** Port **108** may define an opening within seal anchor member **100** having an initial open state. Alternatively, **D_{P1}** may be about **0**mm such that the escape of insufflation gas (not shown) through port **108** of seal anchor member **100** in the absence of surgical object **"I"** is substantially inhibited. For example, port **108** may be a slit extending the length **"L"** of seal anchor member **100** through proximal and distal ends **102, 104.**

Upon the introduction of surgical object **"I",** port **108** transitions to a second slate in which port **108** defines a second, larger dimension **D_{P2}** that substantially approximates the diameter **D₁** of surgical object **"I"** such that a substantially fluid-tight seal is formed therewith, thereby substantially inhibiting the escape of insufflation gas (not shown) through port **108** of seal anchor member **100** in the presence of surgical object **"I". D₁,** and thus **D_{P2}**, will generally lie within the range of about **5**mm to about **12**mm, as these dimensions are typical of the surgical objects used during the course of minimally invasive procedures. However, a seal anchor member **100** including a port **108** that is capable of exhibiting substantially larger, or smaller, dimensions in the second state thereof is not beyond the scope of the present disclosure. Seal anchor member **100** may include a plurality of generally tubular port segments (not shown) defining ports **108.** In addition, seal anchor **100** may be devoid of ports **108.** With this arrangement, ports **108** are created within seal anchor member **100** during the insertion of the surgical object **"I".** In accordance with this embodiment, seal anchor member **100** is formed of a flowable or sufficiently compliant material such as a foam material, e.g., an open-cell polyurethane foam, or a gel.

Ports **108** may include ports **108a,** which contain at least one undercut **118** that collects insufflation gas that leaks through the substantially fluid-light seal between a surgical instrument **"I"** and a port **108a.** Each undercut **118** defines a diameter **D_{P3}** greater than **D_{P2}** and a length along a port **108a** less than **"L".** Insufflation gas that leaks through a substantially fluid-tight seal between an instrument **"I"** and a port **108a** may collect in an undercut **118** to inhibit further leakage of the gas through the substantially fluid-tight seal. Furthermore, the undercuts **118** provide edges (where the respective diameters **D_{P1}** of the lumen **108** transition to the diameters **D_{P3}** of the undercut **118**) that engage the outer surfaces of the instruments **"I"** inserted therethrough to further reduce leakage. Ports **108** may also include ports **108b,** which do not contain undercuts **118,** or any combination of ports **108a** and ports **108b.**

Generally, ports **108** are arranged linearly with respect to major diameter **D₁.** Ports **108** may alternatively be arranged linearly with respect to major diameter **D₂** or dimension **"R".** However, embodiments in which ports **108** are arranged nonlinearly, e.g., an oval or zigzag pattern, are also within the scope of this disclosure. Each port **108** may be spaced equally from its neighboring ports. However, embodiments in which ports **108** are spaced unequally are also within the scope of this disclosure.

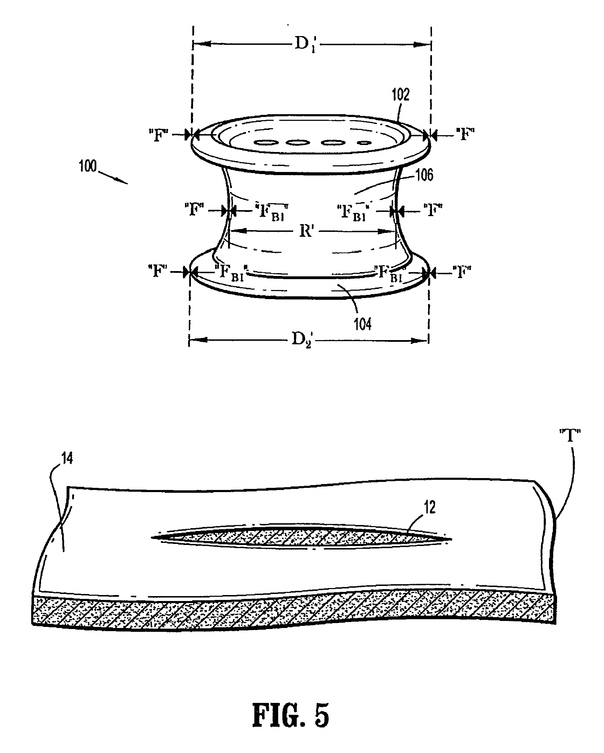

Referring now to **FIGS. 1** and **5****,** seal anchor member **100** is adapted to transition from an expanded condition **(****FIG. 1****)** to a compressed condition **(****FIG. 5****)** so as to facilitate the insertion and securement thereof within tissue tract **12** in tissue **"T".** In the expanded condition, seal anchor member **100** is at rest and the respective major axes **D₁, D₂** of the proximal and distal ends **102,104** of seal anchor member **100,** as well as the dimension **"R"** of the intermediate portion 106 are such that the seal anchor member **100** cannot be inserted within tissue tract **12.** However, as seen in **FIG. 5****,** in the compressed condition, proximal and distal ends **102, 104** of seal anchor member **100** as well as intermediate portion **106** are dimensioned for insertion into tissue tract **12.**

Seal anchor member **100** is formed of a biocompatible compressible material that facilitates the resilient, reciprocal transitioning of seal anchor member **100** between the expanded and compressed conditions thereof. In one embodiment, the compressible material is a "memory" foam. An external force **"F"** is applied to seal anchor member **100** to cause the seal anchor member **100** to assume the compressed condition. External force **"F"** is directed inwardly and when seal anchor member **100** is subjected thereto, e.g., when seal anchor member **100** is squeezed, seal anchor member **100** undergoes an appreciable measure of deformation, thereby transitioning into the compressed condition.

As depicted in **FIG. 5****,** as seal anchor member **100** is compressed under the influence of external force "F" an internal biasing force **"F_{B1}"** is created within seal anchor member **100** that is directed outwardly, opposing force **"F".** Internal biasing force **"F_{B1}"** endeavors to expand seal anchor member **100** and thereby return seal anchor member **100** towards the expanded condition thereof Accordingly, as long as seal anchor member **100** is subject to external force **"F"** greater than biasing force **"F_{B1}",** seal anchor member **100** is compressed, and. once compressed, as long as external force **"F"** at least equals biasing force **"F_{B1}",** seal anchor member **100** remains in the compressed condition. Upon the removal of external force **"F"** biasing force **"F_{B1}"** acts to return seal anchor member **100** towards the expanded condition.

The compressible material comprising seal anchor member 100 also facilitates the resilient transitioning of port **108** between its first state **(****FIGS. 1-3****)** and its second state **(****FIG. 5****).** As previously discussed, prior to the insertion of surgical object **"I",** port **108** is in its first state in which port **108** defines a first or initial dimension **D_{P1}.** Port **108** may incorporate a slit extending the length "L" of seal anchor member **100.** In this first state, port **108** is at rest and is not subject to any external forces. However, upon the introduction of surgical object **"I"** through port **108** as depicted in **FIG. 4****,** the surgical object **"I"** exerts a force **"F₁"** upon port **108** that is directed radially outward. Force **"F₁"** acts to enlarge the dimensions of port **108** and thereby transition port **108** into the second stale thereof in which port **108** defines a second, larger dimension **D_{P2}** that substantially approximates the diameter **D₁** of surgical object **"I".** Consequently, an internal biasing force **"F_{B2}"** is created that is directed radially inward, in opposition to force **"F₁".** Internal biasing force **"F_{B2}"** endeavors to return port **108** to reduce the internal dimension of port **108** and thereby return port **108** to the first state thereof Internal biasing force **"F_{B2}"** is exerted upon surgical object **"I"** and acts to create a substantially fluid-tight seal therewith. The significance of forces **"F_{B1}"** and **"F_{B2}"** will be discussed in further detail below.

It should be noted that, according to various embodiments of the present invention, a particular advantage of the seal anchor member **100** may be that, when the seal anchor member **100** is positioned within an elongated incision, the biasing force exerted by the seal anchor member **100** against the incision is substantially equal around a perimeter of the seal anchor member **100.** For example, many incisions made during surgery are formed by a surgeon cutting through skin and/or tissue with a scalpel, which generally forms an incision shaped like a slit. This slit-shaped incision is elongated, e.g., relatively narrow in one direction and relatively long in the perpendicular direction - thus, a seal anchor member that has a circular cross-section is required to be more compressed in one direction than in another direction in order to be inserted within an elongated incision, making such a seal anchor member more difficult to insert within the incision. Additionally, once a seal anchor member with a circular cross-section is positioned within the incision, such a seal anchor member may generate different biasing forces against the incision at various positions around its perimeter. This may cause the sealing effect between such a seal anchor member to be different at various positions around the seal anchor member's perimeter and may result in an increased likelihood of leakage, e.g., at the furthermost ends of the elongated incision where the seal anchor member has experienced the least amount of compression and thus is exerting the least amount of biasing force against the incision. In contrast, an elongated seal anchor member, e.g., that has a major diameter that is greater than a minor diameter, may advantageously be compressed a substantially equal amount in all directions in order to be inserted within an elongated incision, thus easing its insertion. Furthermore, once an elongated seal anchor member is positioned within the incision, the elongated seal anchor member may generate substantially equal biasing forces against the incision around its entire perimeter. This may result in a more constant sealing effect between the seal anchor member and the incision at all of the various positions around the seal anchor member's perimeter, thereby reducing the likelihood of leakage, particularly at the furthermost ends of the elongated incision where conventional seal anchor member have typically experienced the greatest likelihood of leakage.

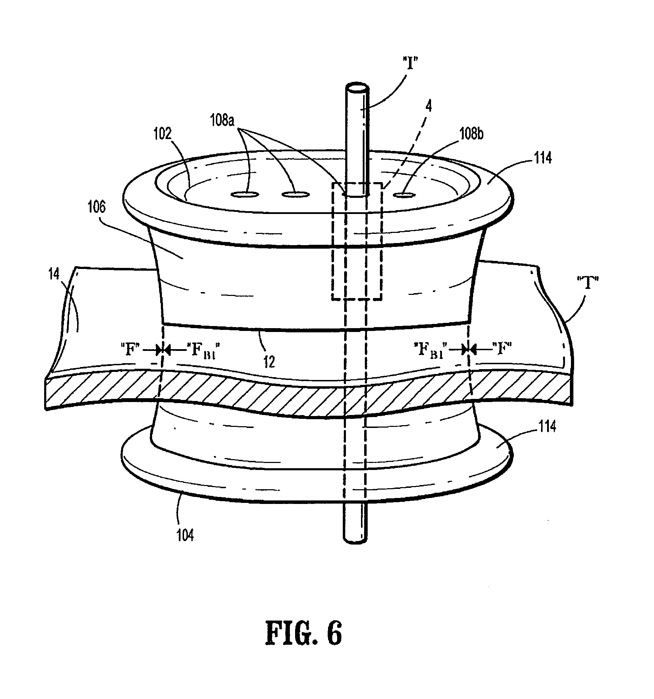

Referring again to **FIG. 1**, one or more positioning members **114** may be associated with either or both of proximal end **102** and distal end **104** of seal anchor member **100.** Positioning members **114** may be composed of any suitable biocompatible material that is at least semi-resilient such that positioning members **114** may be resiliency deformed and may exhibit any suitable elongated configuration, e.g., substantially oblong or oval. Prior to the insertion of seal anchor member **100,** positioning members **114** are deformed in conjunction with the respective proximal and distal ends **102, 104** of seal anchor member **100** to facilitate the advancement thereof through tissue tract **12 (****FIG. 6****).** Subsequent to the insertion of seal anchor member **100** within tissue tract **12,** the resilient nature of positioning members **114** allows positioning members to return towards their normal, e.g., substantially oblong or oval, configuration, thereby aiding in the expansion of either or both of the respective proximal and distal ends **102, 104** and facilitating the transition of seal anchor member **100** from its compressed condition to its expanded condition. Positioning members **114** also may engage the walls defining the body cavity to further facilitate securement of seal anchor member **100** within the body tissue. For example, positioning member **114** at leading end **104** may engage the internal peritoneal wall and positioning member **114** adjacent trailing end **102** may engage the outer epidermal tissue adjacent the incision **12** within tissue **"T".** In another embodiment of seal anchor member **100,** one or more additional positioning members **114** may be associated with intermediate portion **106.**

The use of seal anchor member **100** will be discussed during the course of a typical minimally invasive procedure. Initially, the peritoneal cavity (not shown) is insufflated with a suitable biocompatible gas, such as CO₂ gas, such that the cavity wall is raised and lifted away from the internal organs and tissue housed therein, providing greater access thereto. The insufflation may be performed with an insufflation needle or similar device, as is conventional in the art. Either prior or subsequent to insufflation, a tissue tract **12** is created in tissue **"T",** the dimensions of which may be varied dependent upon the nature of the procedure.

Prior to the insertion of seal anchor member **100** within tissue tract **12,** seal anchor member **100** is in its expanded condition in which the dimensions thereof prohibit the insertion of seal anchor member **100** into tissue tract **12.** To facilitate insertion, the clinician transitions seal anchor member **100** into the compressed condition by applying a force **"F"** thereto, e.g., by squeezing seal anchor member **100.** Force **"F"** acts to reduce the dimensions **D₁** and **D₂** of the proximal and distal ends **102, 104,** respectively, to **D₁'** and **D₂' (****FIG. 5****)** including positioning members **114** (if provided) and to reduce the dimension **"R"** of intermediate portion **106** to **"R'"** such that seal anchor member **100** may be inserted into tissue tract **12.** As best depicted in **FIG. 6****,** subsequent to its insertion, distal end **104,** positioning member **114** (if provided), and at least a section **112** of intermediate portion **106** are disposed beneath the tissue **"T".** Seal anchor member **100** is caused to transition from the compressed condition to the expanded condition by removing force **"F"** therefrom.

During the transition from the compressed condition to the expanded condition, the dimensions of seal anchor member **100,** i.e., the respective dimensions **D₁', D2' (****FIG. 5****)** of the proximal and distal ends **102, 104** are increased towards **D₁** and **D₂ (****FIG. 6****)** and the dimension **"R"'** is increased towards **"R".** The expansion of distal end 104 is relatively uninhibited given the disposition thereof beneath tissue **"T",** and accordingly, distal end **104** is permitted to expand substantially, if not completely. However, as seen in **FIG.** 5, the expansion of the section **112** of the intermediate portion **106** is limited by the tissue surfaces **14 (****FIG. 1****)** defining tissue tract **12,** thereby subjecting intermediate portion **106** to an external force **"F"** that is directed inwardly. As discussed above, this creates an internal biasing force **"F_{B1}"** that is directed outwardly and exerted upon tissue surfaces **14,** thereby creating a substantially fluid-tight seal between the seal anchor member **100** and tissue surfaces **14** and substantially inhibiting the escape of insufflation gas around seal anchor member **100** and through tissue tract **12.**

In the expanded condition, the respective dimensions **D₁, D₂** of the proximal and distal ends **102, 104** are larger than the dimension **"R"** of the intermediate portion **106.** Subsequent to insertion, the dimension **D₂** of distal end **104** and positioning member **114** is also substantially larger than the dimensions of the tissue tract **12.** Consequently, seal anchor member **100** may not be removed from tissue tract **12** in the expanded condition and thus, seal anchor member **100** will remain anchored within the tissue "T" until it is returned to its compressed condition.

After successfully anchoring seal anchor member **100** within the patient's tissue **"T",** one or more surgical objects **"I"** may be inserted through ports **108.** **FIG. 6** illustrates a surgical object **"I"** introduced through one of ports **108.** As previously discussed, prior to the insertion of surgical object **"I",** port **108** is in its first slate in which port **108** defines an initial dimension **D_{P1}** which may be negligible in that port **108,** in one embodiment, is a slit. Accordingly, prior to the escape of insufflation gas through port **108,** in the absence of surgical object **"I"** is minimal, thereby preserving the integrity of the insufflated workspace.

Surgical object **"I"** may be any suitable surgical instrument and, accordingly, may vary in size. Suitable surgical objects to be introduced within one or more of the ports 108 include minimally invasive grasper instruments, forceps, clip-appliers, staplers, cannula assemblies, etc. Upon the introduction of surgical object **"I",** port **108** is enlarged, thereby transitioning into its second state in which port **108** defines a second dimension **D_{P2} (****FIG. 4****)** that substantially approximates the diameter **D₁** of surgical object **"I",** thereby creating a substantially fluid-tight seal with surgical object "I" and substantially inhibiting the escape of insufflation gas (not shown) through port **108** of seal anchor member **100** in the presence of a surgical object **"I",** as previously discussed.

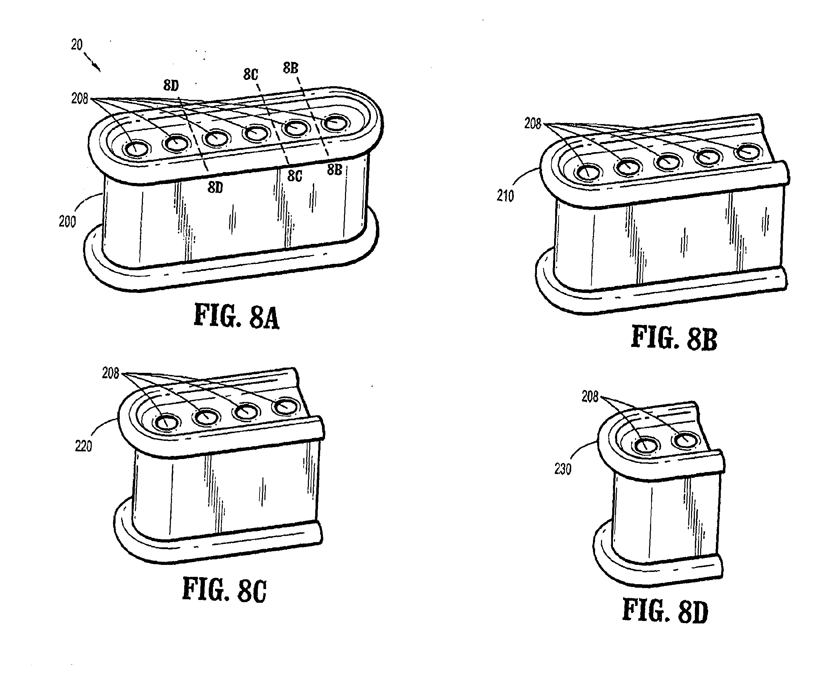

Turning now to **FIGS. 8A-8D****,** a surgical apparatus, in accordance with an alternate embodiment of the present disclosure, is generally designated as **20.** Surgical apparatus **20** is substantially identical to surgical apparatus **10** and thus will only be discussed in detail herein to the extent necessary to identify differences in construction and operation thereof.

As seen in **FIG. 8A****,** surgical apparatus **20** comprises a seal anchor member **200** defining a plurality of ports **208.** If seal anchor member **200** defines more ports **208** than are required for a particular surgical procedure, seal anchor member **200** may be cut to have a fewer number of ports **208.** **FIGS. 8B-8D** illustrate resulting seal anchor members **210, 220,** and **230** when seal anchor member **200** is cut along segment lines **8B-8B, 8C-8C,** and **8D-8D** respectively. Seal anchor member **200** may include indicia, e.g., lines or markings along segment lines **8B-8B, 8C-8C,** and **8D-8D,** etc., that indicate to a user a location at which to make such a cut if desired. Additional or alternatively, the seal anchor member **200** may include a weakened region, e.g., perforations, slits, etc., at such locations that facilitate or ease the making of such a cut. Seal anchor member **200** and resulting seal anchor members **210, 220,** and **230,** may be used in a surgical procedure in a substantially similar manner to seal anchor member **100** as discussed hereinbefore.

As set forth above, the prevent invention, according to various embodiments thereof, may provide particular advantages for, e.g., thoracic procedures (for example, thymectomies, lobectomies, pneumonectomy, esophagectomy, mediastinal tumor resection, sympathectomy, etc.) and/or single incision laparoscopic procedures in which it may be desirable to access an abdominal cavity off-midline. For example, during thoracic procedures, access is typically attained by placing cannulas or instruments between a patient's ribs. The elongated shape, when viewed in cross-section, of the seal anchor member, along with the linear arrangement of the ports therethrough, allows the seal anchor member to be inserted between a patient's ribs and to move with the natural curvature of the ribeage. In single incision laparoscopic procedures, the shape of the seal anchor member may enable it to be positioned between muscle groups, e.g., parallel to and on the lateral edge of the rectus abdominus muscles. Advantageous positioning of the seal anchor member, as described hereinabove, may provide additional benefits of reducing stretching, trauma and post-operative pain.

In some instances, thoracic procedures may not require insufflation. For other types of surgical procedures, e.g., laparoscopic procedures, insufflation may be used - for these types of procedures, the seal anchor member may be provided with insufflation tubing (not shown) or one of the ports may be specifically employed for insufflation purposes.

Although the illustrative embodiments of the present disclosure have been described herein with reference to the accompanying drawings, the above description, disclosure, and figures should not be construed as limiting, but merely as exemplifications of particular embodiments. It is to be understood, therefore, that the disclosure is not limited to those precise embodiments, and that various other changes and modifications may be effected therein by one skilled in the art without departing from the scope or spirit of the disclosure.

## Claims

1. A surgical apparatus for positioning within a tissue tract accessing an underlying body cavity, which comprises:
a seal anchor member comprising a compressible material;
the seal anchor member being adapted to transition between a first condition for insertion of at least a portion of the seal anchor member within a tissue tract and a second condition to facilitate a securing of the seal anchor member within a tissue tract and in substantial sealed relation with tissue surfaces defining a tissue tract,
the seal anchor member having proximal and distal ends and defining at least one port extending between the proximal and distal ends, the at least one port being adapted for the reception of an object whereby compressible material defining the at least one port is adapted to deform to establish a substantial scaled relation with the object, and
wherein the seal anchor member has a non-circular cross-section.

2. The surgical apparatus according to claim 1, wherein the seal anchor member is formed of a foam material.

3. The surgical apparatus according to claim 2, wherein the foam material is at least partially constituted of a material selected from the group consisting of polyisoprene, urethane, and silicone.

4. The surgical apparatus according to claim 1, wherein the seal anchor member is formed of a gel material.

5. The surgical apparatus according to any preceding claim, wherein the at least one port includes at least one undercut to reduce the likelihood of leaks therethrough.

6. The surgical apparatus according to any preceding claim, wherein the surgical apparatus includes indicia that indicates to a user a location at which the apparatus may be cut.

7. The surgical apparatus according to any preceding claim, wherein the seal anchor member includes a plurality of ports.

8. The surgical apparatus according lo claim 7, wherein the plurality of ports is configured linearly with respect to each other.

9. The surgical apparatus according to claim 7, wherein each port of the plurality of ports is spaced equally from its neighboring ports.

10. A seal anchor member formed from a foam material and being elongated when viewed in cross-section, the member being configured and dimensioned to be compressed for insertion into an incision and, once inserted, to expand so as to be secured within and seal against the incision, the seal anchor member defining at least one port extending generally longitudinally and being adapted for sealed reception of a surgical object.

11. The seal anchor member according to claim 10, wherein, when the seal anchor member is positioned within an incision, the seal anchor member exerts a biasing force against the incision.

12. The seal anchor member according to claim 11, wherein, when the seal anchor member is positioned within an elongated incision, the biasing force exerted by the seal anchor member against the incision is substantially equal around a perimeter of the seal anchor member.

13. The seal anchor member according to any of claims 10 to 12, wherein the foam material is at least partially constituted of a material selected from the group consisting of polyisoprene, urethane, and silicone.

14. The seal anchor member according to any of claims 10 to 13, wherein the seal anchor member includes a plurality of ports.

15. A seal anchor member formed from a foam material, the member being configured and dimensioned to be compressed for insertion into an elongated incision and, once inserted, to expand so as to exert a biasing force against the elongated incision, the seal anchor member having a shape in cross-section such that the biasing force exerted by the seal anchor member against the elongated incision is substantially equal around a perimeter of the seal anchor member.

16. The seal anchor member according to claim 15, the seal anchor member defining at least one port extending generally longitudinally and being adapted for sealed reception of a surgical object.

17. The seal anchor member according to claim 15 or claim 16, wherein the foam material is at least partially constituted of a material selected from the group consisting of polyisoprene, urethane, and silicone.

18. The seal anchor member according to any of claims 15 to 17, wherein the seal anchor member includes a plurality of ports.

19. The seal anchor member according to any of claims 15 to 18, wherein the seal anchor member defines a major diameter in a first direction and a minor diameter in a second direction that is perpendicular to the first direction, the major diameter being greater than the minor diameter, preferably wherein the seal anchor member includes an intermediate portion and an end portion, at least one of a major diameter and a minor diameter of the end portion being greater than a major diameter or a minor diameter of the intermediate portion so as to aid in retaining the seal anchor member within an incision.
